# EUROPEAN PATENT APPLICATION

(11) **EP 4 410 284 A1**
(43) Date of publication of application: **07.08.2024**
(21) Application number: 23305135.8
(22) Date of filing: 02.02.2023
(51) Int. Cl.: A61K 31/18, A61K 31/27, A61P 43/00

(54) **COMPOUND AND METHOD FOR THE TREATMENT OF ZELLWEGER SPECTRUM DISORDER**

(71) Applicant: Apteeus, 59000 Lille (FR)
(72) Inventor: BELLOY, Loïc, 59263 HOUPLIN-ANCOISNE (FR); MOREAU-VANBESIEN, Camille, 59280 ARMENTIERES (FR); BEGHYN, Terence, 59320 HAUBOURDIN (FR)
(74) Representative: Cabinet Beau de Loménie

(57) **Abstract**

The present disclosure relates to the use of a histone deacetylase, or of a pharmaceutical composition containing such an inhibitor, for the prevention or treatment of a peroxisomal biogenesis disorder.

## Description

### FIELD OF THE DISCLOSURE

The present disclosure relates to compounds useful for the treatment of Zellweger Spectrum Disorder, and to compositions containing such compounds.

### STATE OF THE ART

Zellweger Spectrum Disorder (ZSD), also known as Peroxisome biogenesis disorder (PBD), is a group of rare genetic autosomal recessive diseases caused by peroxin (PEX) gene mutations that result in abnormal peroxisome assembly, structure, and function. The Zellweger Spectrum represents a clinical continuum, with Zellweger syndrome (ZS) having the most severe phenotype, and neonatal adrenoleukodystrophy (NALD) and infantile Refsum disease (IRD) having progressively milder phenotypes. Rhizomelic chondrodysplasia punctata (RCDP) spectrum are a group of rare disorders that are also classified as peroxisomal biogenesis disorders.

Neurological deficits, loss of muscle tone (hypotonia), hearing loss, vision problems, liver dysfunction, and kidney abnormalities are common findings. ZSD often result in severe, life-threatening complications early during infancy. Some individuals with milder forms have lived into adulthood. ZSD are inherited in an autosomal recessive pattern.

Although no treatment is currently available for ZSD, it has recently been suggested that small molecules could be effective in restoring peroxisomes assembly and function in PEX mutated fibroblasts¹⁻³. However, there is still a need to find therapeutical approaches for treating this kind of disease by addressing the peroxisome assembly defects responsible for disease development and progression.

ZSD develops due to mutations of one of 13 different genes involved in the creation and proper function of peroxisomes (peroxisome biogenesis). These 13 genes contain instructions for encoding proteins known as peroxins (PEX) that are essential for the proper development of peroxisomes. Among ZSD patients, PEX1 (58%) is the most affected gene followed by PEX6 (16%) and PEX12 (9%)⁴. PEX26 mutations account for only 3% of total ZSD⁴. The identified PEX mutations comprise insertions, deletions, nonsense, missense, and splice site mutations⁵. There are two predominant PEX1 gene mutations found in people with Zellweger Spectrum Disorder. The first one is Gly843Asp (or G843D) in PEX1p. This mutation leads to reduced levels of the protein. Individuals who have the G843D mutation tend to have signs and symptoms that are at the less-severe end of the condition spectrum. The other predominant mutation is the I700fs mutation (or [Ile700Tyrfs42^{∗}]), which leads to the production of an abnormally short, nonfunctional PEX1p. People who have the I700fs mutation often have signs and symptoms that are at the severe end of the condition spectrum. Studies on more than 800 patients show that defects in PEX1 represent around 60% of all ZSD alleles. In these reports, 12-30% of ZSD alleles are PEX1-p.G843D, and 5-20% are PEX1 p.[Ile700Tyrfs42^{∗}]⁶.

Some genetic mutations may result in protein instability. Depending on the reasons of such instability, several strategies have been explored using small molecules to stabilize the defective protein. Evidence in support of those strategies has been reported in the literature: see Fiedorczuck et Chen (2022)⁷; Galafold Epar Product Information (EMEA/H/C/00405059); Alfonso et al. (2005)⁸ ; Porto et al. (2009)⁹; Kirkegaard. et al. (2016)¹⁰; Fog et al. (2018)¹¹; Maegawa et al. (2009)¹²; Hutt et al. (2010)¹³; Anglès et al. (2019)¹⁴; Lu et al. (2011)¹⁵ ; Pipalia et al. (2017)¹⁶ ; Angelini et al. (2022)¹⁷; or Bartoli et al. (2008)¹⁸.

Several publications mentioned the possibility to restore peroxisomes biogenesis and function in PEX1 deficient cells resulting from nonsense mutations¹⁹ and missense mutations, mostly the PEX1-p.G843D mutation, with chemical chaperones such as Glycerol, Betaine, Arginine, 4-phenylbutyrate or flavonoids such as Diosmetin ^{1-3,20}. US patent application 2021/315885 A1 discloses a method of treating ZSD comprising administering to a subject in need thereof a compound of formula:

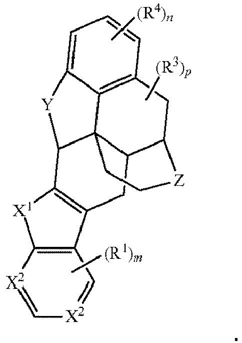

WO 2006/102557 discloses inter alia a method of treating a subject suffering from a protein degradation disorder comprising administering to a subject in need thereof a therapeutically effective amount of a protein degradation inhibitor.

To date, however, there are no authorized therapies for the treatment of ZSD which is a notoriously difficult disease to treat. There is therefore a need to find compounds which are effective to treat ZSD. It has now been found, and this is the gist of the present disclosure, that histone deacetylase (HDAC) inhibitors are useful for treating peroxisome biogenesis disorders resulting from mutations in a PEX gene.

### SUMMARY OF THE INVENTION

The present disclosure relates to the use of histone deacetylase inhibitors in the prevention or treatment of peroxisomal biogenesis disorder (PBD), aka Zellweger Spectrum Disorder (ZSD), and to pharmaceutical compositions comprising said inhibitors. The present disclosure also relates to a method of preventing or treating peroxisomal biogenesis disorder which comprises administering a histone deacetylase inhibitor to a subject in need thereof.

In some embodiments, the histone deacetylase inhibitor is selected from the group consisting of: Oxamflatin, Quisinostat, Belinostat, Pracinostat, Trichostatin A, Dacinostat, Nanatinostat, LMK-235, Panobinostat, FNDR-20123, QTX125 (TFA), Givinostat (hydrochloride monohydrate), Fimepinostat, M344, Nampt-IN-3, HDACs/mTOR Inhibitor 1, ACY-957, CAY10603, BRD-6929, Mocetinostat, CUDC-101, Resminostat (hydrochloride), Corin, Scriptaid, CXD101, HDAC-IN-3, JAK/HDAC-IN-1, Citarinostat, Entinostat, Tucidinostat, Vorinostat, Nexturastat A, EDO-S101, Tefinostat, Splitomicin, ACY-738, Ricolinostat, J22352, HPOB, MPT0G211, NKL 22, Tubastatin A, SR-4370, Bufexamac, CG347B, Pyroxamide, MC1568, Santacruzamate A, BML-210, Tasquinimod, Pimelic Diphenylamide 106, AES-350, BRD 4354, ACY-775, Domatinostat, SW-100, AES-135, Sinapinic acid, Marein, Crotonoside, WT-161, BRD73954, Ac-Lys-AMC, KA2507, RG2833, ACY-1083, PCI-34051, BG45, Tacedinaline, FT895, Suberoyl bis-hydroxamic acid, TMP195, Remetinostat, Tubastatin A (Hydrochloride), Droxinostat, Gnetol, 1-Naphthohydroxamic acid, BRD3308, UF010, TMP269, TH34, RGFP966, HDAC8-IN-1, CHDI-390576 and ITSA-1. In some embodiments, the histone deacetylase inhibitor is selected from the group consisting of: Quisinostat, Belinostat, Pracinostat, Nanatinostat, Panobinostat, Givinostat (hydrochloride monohydrate), Fimepinostat, Mocetinostat, CUDC-101, Resminostat (hydrochloride), CXD101, Citarinostat, Entinostat, Tucidinostat, Vorinostat, EDO-S101, Tefinostat, Ricolinostat, Domatinostat and KA2507.

In some embodiments, peroxisomal biogenesis disorder results from a mutations in a PEX gene.

### BRIEF DESCRIPTION OF THE DRAWINGS

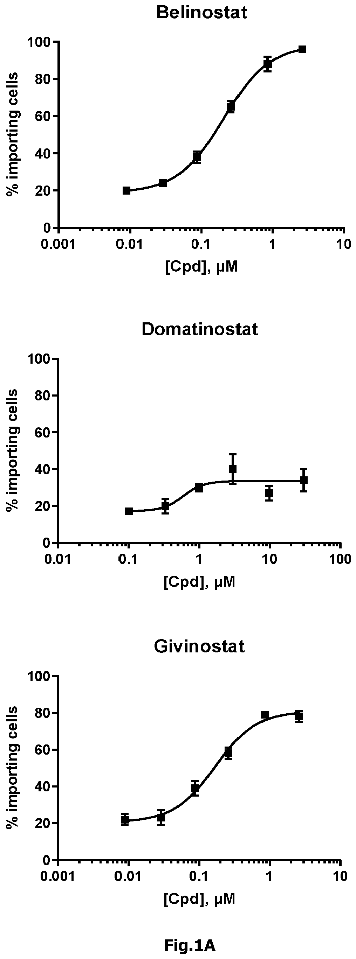

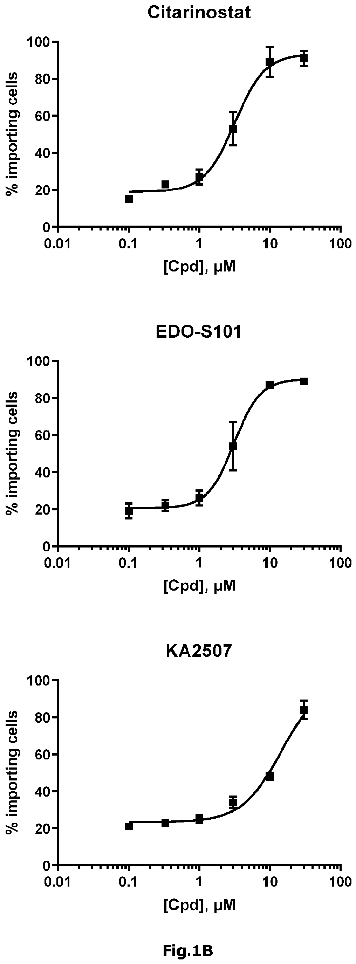

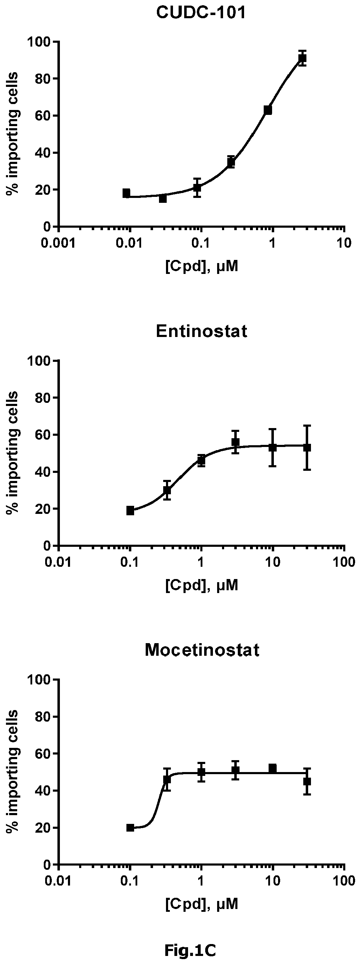

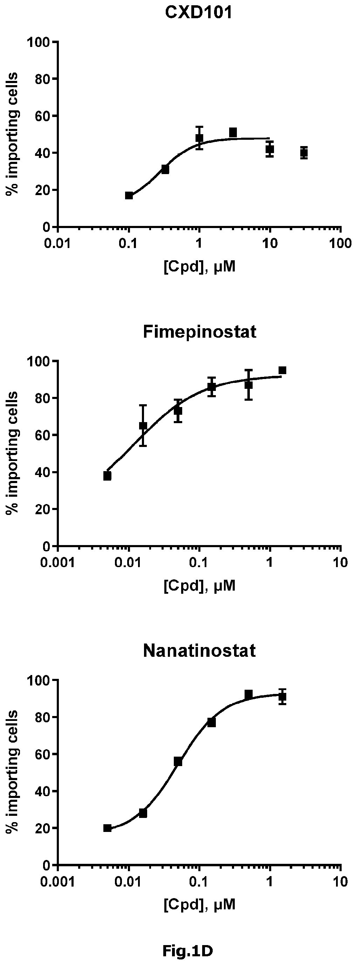

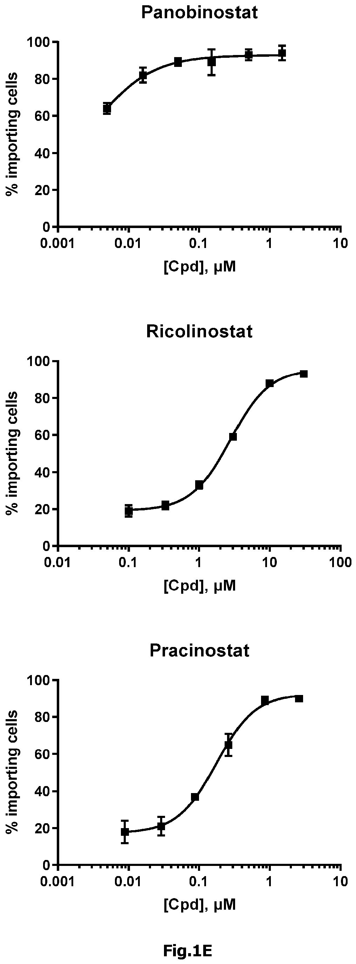

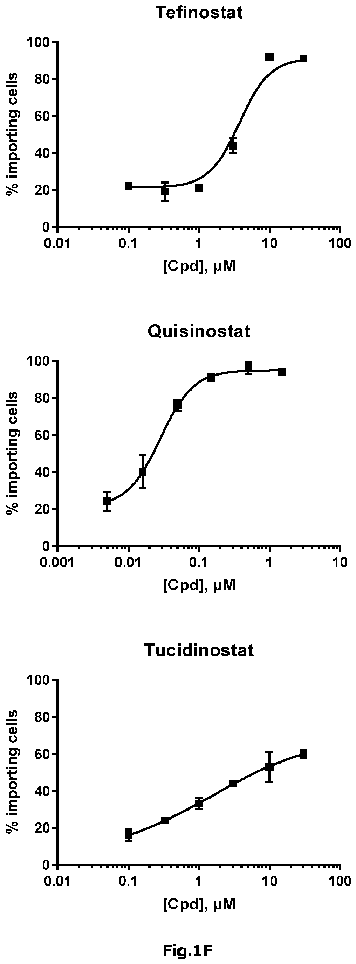

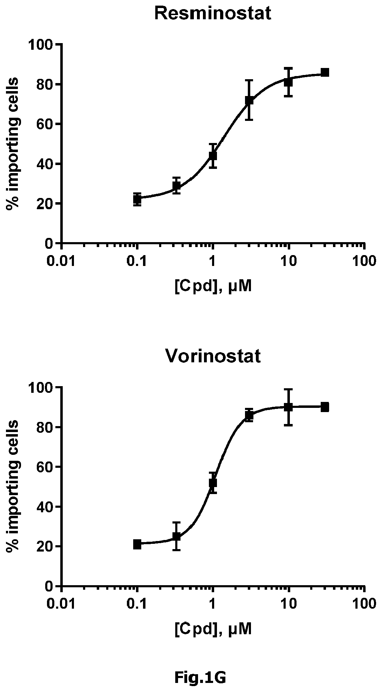

Figures 1A to 1G represent the dose response curve of the percentage of catalase importing GM16513 cells treated for 3 days with representative HDAC inhibitors of the present disclosure.

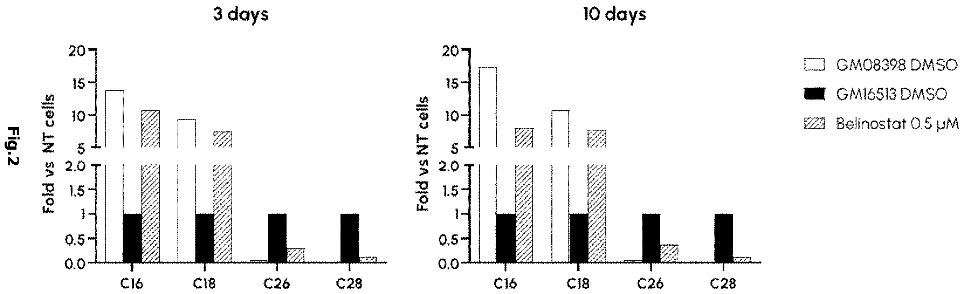

Figure 2 represents the effect of a 3- or 10-day treatment with Belinostat at 0.5 µM on β-oxidation in GM1513 cells. Cells were cultured in a medium containing C24:0-d4 over 3 days before cells harvesting. The d4-LCFA and d4-VLCFA levels were normalized by the level in GM16513 non treated cells (fold vs NT).

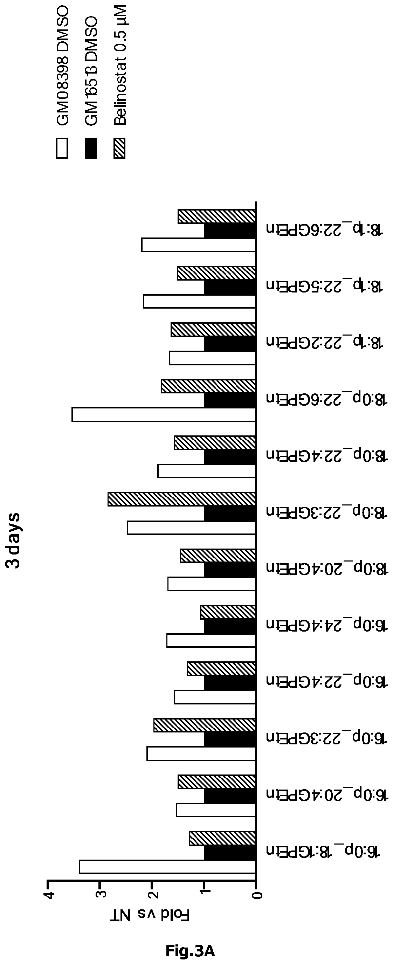

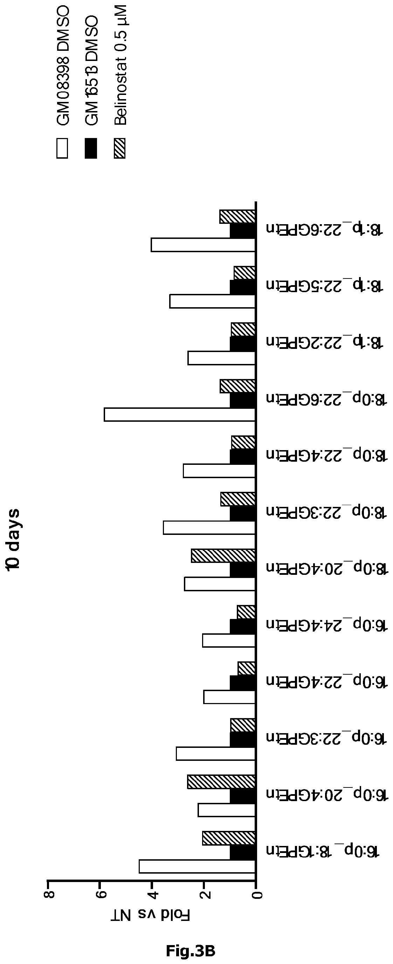

Figures 3A and 3B respectively represent the effect of a 3- or 10-day treatment with Belinostat at 0.5 µM on plasmalogens synthesis in GM16513 cells. Only those plasmalogens with a sufficient signal to be analyzed and which show a lower quantity in comparison to GM08398 cells (Healthy control cells) are represented. Plasmalogens levels were normalized by the level in GM16513 non treated cells (fold vs NT).

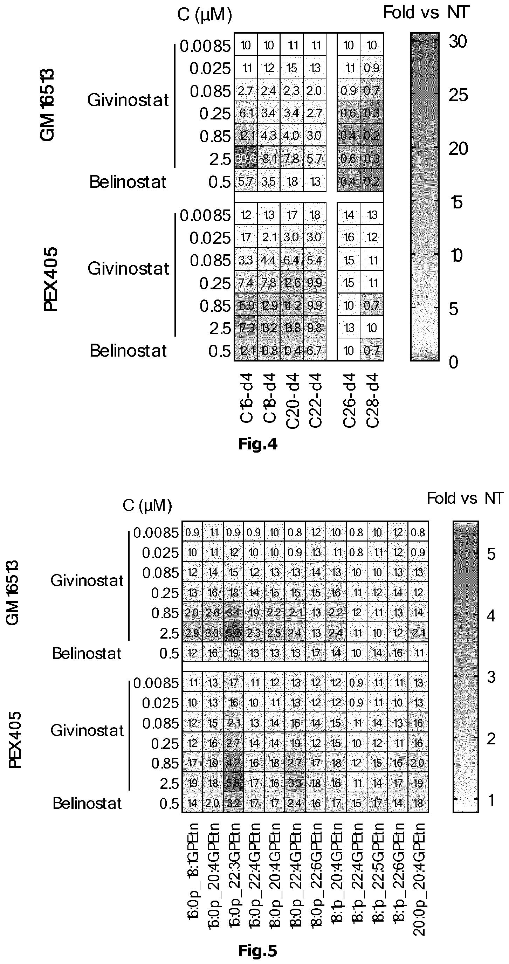

Figure 4 represents a heatmap showing the effect of a 3-day treatment with Belinostat and Givinostat on β-oxidation in GM16513 and PEX405 cells. Cells were cultured in a medium containing C24:0-d4 over 3 days before cells harvesting. The d4-LCFA and d4-VLCFA levels were normalized by the level in GM16513 or PEX405 non treated cells (fold vs NT). Mean of 2 independent experiments.
Figure 5 represents a heatmap showing the effect of a 3-day treatment with Belinostat and Givinostat on plasmalogens synthesis in GM16513 and PEX405 cells. Only those plasmalogens with a sufficient signal to be analyzed and which show a lower quantity in comparison to GM08398 cells (Healthy control cells) are represented. Plasmalogens levels were normalized by the level in GM16513 or PEX405 non treated cells (fold vs NT). Mean of 3 independent experiments.

### DETAILED DESCRIPTION OF THE INVENTION

The present disclosure relates to the use of a histone deacetylase (HDAC) inhibitor, or of a pharmaceutical composition comprising such an inhibitor, in the prevention or treatment of peroxisomal biogenesis disorder (PBD), aka Zellweger Spectrum Disorder (ZSD).

A library of histone deacetylase (HDAC) inhibitors was screened according to the procedure described in the examples (section "Materials and methods"). In short, the cytotoxicity of all inhibitors was assessed in parallel on GM16513 and PEX405 cells using a double nucleus staining with Hoechst 33342 and Propidium Iodide (PI). The % of importing cells and % of PI positive cells (dead cells) are shown in tables 1 and 2.

**Table 1**

| | **GM16513 cells** | | | |
|---|---|---|---|---|
| **Molecule Name** | **% Importing cells** | **% PI+ cells** | **% Importing cells** | **% PI+ cells** |
| DMSO 0.1% | 3 | 1 | 3 | 1 |

| | **1.3 µM** | | **10 µM** | |
|---|---|---|---|---|
| Oxamflatin | 99 | 5 | 59 | 18 |
| Quisinostat | 98 | 17 | 86 | 38 |
| Belinostat | 95 | 4 | 97 | 21 |
| Pracinostat | 92 | 4 | 86 | 31 |
| Trichostatin A | 90 | 18 | 76 | 30 |
| Dacinostat | 88 | 31 | 76 | 35 |
| Nanatinostat | 88 | 12 | 78 | 37 |
| LMK-235 | 86 | 2 | 95 | 15 |
| Panobinostat | 86 | 30 | 44 | 32 |
| FNDR-20123 | 84 | 11 | 47 | 40 |
| QTX125 (TFA) | 83 | 11 | 61 | 30 |
| Givinostat (hydrochloride monohydrate) | 81 | 6 | 32 | 29 |
| Fimepinostat | 81 | 25 | 61 | 29 |
| M344 | 80 | 2 | 85 | 12 |
| Nampt-IN-3 | 80 | 3 | 74 | 3 |
| HDACs/mTOR Inhibitor 1 | 77 | 14 | 96 | 24 |
| ACY-957 | 74 | 1 | 0 | 38 |
| CAY10603 | 73 | 2 | 85 | 31 |
| BRD-6929 | 66 | 1 | 88 | 3 |
| Moceti nostat | 60 | 4 | 39 | 9 |
| CUDC-101 | 60 | 2 | 90 | 23 |
| Resminostat (hydrochloride) | 60 | 2 | 83 | 8 |

| | **GM16513 cells** | | | |
|---|---|---|---|---|
| **Molecule Name** | **% Importing cells** | **% PI+ cells** | **% Importing cells** | **% PI+ cells** |
| Corin | 59 | 5 | 44 | 34 |
| Scriptaid | 57 | 2 | 92 | 8 |
| CXD101 | 56 | 2 | 50 | 7 |
| HDAC-IN-3 | 50 | 3 | 81 | 26 |
| JAK/HDAC-IN-1 | 49 | 2 | 64 | 23 |
| Citarinostat | 47 | 1 | 90 | 4 |
| Entinostat | 45 | 2 | 58 | 7 |
| Tucidinostat | 43 | 1 | 64 | 4 |
| Vorinostat | 35 | 2 | 96 | 9 |
| Nexturastat A | 35 | 2 | 83 | 4 |
| EDO-S101 | 35 | 2 | 89 | 8 |
| Tefi nostat | 33 | 2 | 91 | 2 |
| Splitomicin | 33 | 1 | 25 | 1 |
| ACY-738 | 31 | 1 | 85 | 3 |
| Ricolinostat | 30 | 2 | 89 | 5 |
| J22352 | 30 | 1 | 47 | 3 |
| HPOB | 29 | 1 | 55 | 1 |
| MPT0G211 | 29 | 1 | 65 | 4 |
| NKL 22 | 28 | 1 | 55 | 4 |
| Tubastatin A | 26 | 1 | 32 | 1 |
| SR-4370 | 26 | 1 | 30 | 2 |
| Bufexamac | 26 | 2 | 25 | 1 |
| CG347B | 25 | 1 | 21 | 1 |
| Pyroxamide | 25 | 1 | 11 | 4 |
| MC1568 | 25 | 3 | 21 | 90 |
| Santacruzamate A | 24 | 1 | 25 | 1 |
| BML-210 | 24 | 2 | 73 | 3 |
| Tasquinimod | 23 | 1 | 22 | 1 |
| Pimelic Diphenylamide 106 | 22 | 1 | 52 | 3 |
| AES-350 | 22 | 2 | 45 | 5 |
| BRD 4354 | 21 | 1 | 30 | 3 |
| ACY-775 | 21 | 2 | 47 | 2 |
| Domatinostat | 21 | 6 | 33 | 7 |
| SW-100 | 21 | 1 | 6 | 5 |
| AES-135 | 21 | 2 | 3 | 12 |
| Sinapinic acid | 21 | 1 | 18 | 1 |
| Marein | 20 | 1 | 22 | 77 |
| Crotonoside | 20 | 1 | 18 | 1 |
| WT-161 | 20 | ND | 57 | ND |
| BRD73954 | 19 | 1 | 23 | 1 |
| Ac-Lys-AMC | 18 | 1 | 14 | 1 |
| KA2507 | 17 | 1 | 52 | 2 |
| RG2833 | 17 | 1 | 43 | 3 |
| ACY-1083 | 17 | 2 | 42 | 1 |
| PCI-34051 | 16 | 1 | 7 | 2 |
| BG45 | 15 | 1 | 30 | 1 |
| Tacedinaline | 15 | 1 | 18 | 2 |
| FT895 | 14 | 1 | 4 | 7 |
| Suberoyl bis-hydroxamic acid | 13 | 1 | 57 | 2 |
| TMP195 | 13 | 1 | 8 | 1 |
| Remetinostat | 12 | 1 | 14 | 1 |
| Tubastatin A (Hydrochloride) | 12 | 2 | 37 | 1 |
| Droxinostat | 12 | 1 | 23 | 1 |
| Gnetol | 12 | 1 | 9 | 1 |
| 1-Naphthohydroxamic acid | 12 | 1 | 13 | 1 |
| BRD3308 | 12 | 2 | 16 | 2 |
| UF010 | 12 | 1 | 16 | 1 |
| TMP269 | 11 | 1 | 7 | 1 |
| TH34 | 11 | 1 | 10 | 2 |
| RGFP966 | 11 | 1 | 12 | 3 |
| HDAC8-IN-1 | 10 | 2 | 3 | 14 |
| CHDI-390576 | 10 | 1 | 4 | 2 |
| ITSA-1 | 9 | 1 | 12 | 1 |
| Tubacin | ND | ND | 39 | 4 |

**Table 2**

| | **PEX405 cells** | | | |
|---|---|---|---|---|
| **Molecule Name** | **% Importing cells** | **% PI+ cells** | **% Importing cells** | **% PI+ cells** |
| DMSO 0.1% | 17.5 | 0.5 | 17.5 | 0.5 |

| | **1.3 µM** | | **10 µM** | |
|---|---|---|---|---|
| Oxamflatin | 33 | 2 | 15 | 36 |
| Quisinostat | 40 | 24 | 26 | 53 |
| Belinostat | 52 | 1 | 27 | 42 |
| Pracinostat | 52 | 2 | 25 | 41 |
| Trichostatin A | 28 | 24 | 0 | 49 |
| Dacinostat | 25 | 45 | 0 | 56 |
| Nanatinostat | 14 | 15 | 0 | 54 |
| LMK-235 | 45 | 1 | 18 | 16 |
| Panobinostat | 6 | 48 | 18 | 49 |
| FNDR-20123 | 7 | 9 | 0 | 61 |
| QTX125 (TFA) | 23 | 13 | 0 | 48 |

| | **PEX405 cells** | | | |
|---|---|---|---|---|
| **Molecule Name** | **% Importing cells** | **% PI+ cells** | **% Importing cells** | **% PI+ cells** |
| Givinostat (hydrochloride monohydrate) | 12 | 3 | 6 | 41 |
| Fimepinostat | 25 | 43 | 4 | 55 |
| M344 | 30 | 2 | 11 | 15 |
| Nampt-IN-3 | 33 | 1 | 31 | 4 |
| HDACs/mTOR Inhibitor 1 | 9 | 8 | 33 | 40 |
| ACY-957 | 16 | 1 | 13 | 55 |
| CAY10603 | 22 | 1 | 11 | 35 |
| BRD-6929 | 20 | 1 | 38 | 1 |
| Moceti nostat | 15 | 3 | 9 | 29 |
| CUDC-101 | 23 | 1 | 49 | 44 |
| Resminostat (hydrochloride) | 13 | 1 | 19 | 4 |
| Corin | 17 | 2 | 11 | 66 |
| Scriptaid | 15 | 1 | 16 | 6 |
| CXD101 | 19 | 0 | 4 | 8 |
| HDAC-IN-3 | 11 | 4 | 0 | 43 |
| JAK/HDAC-IN-1 | 13 | 1 | 13 | 29 |
| Citarinostat | 9 | 0 | 53 | 2 |
| Entinostat | 15 | 2 | 12 | 6 |
| Tucidinostat | 14 | 0 | 28 | 2 |
| Vorinostat | 13 | 1 | 16 | 9 |
| Nexturastat A | 8 | 1 | 27 | 3 |
| EDO-S101 | 7 | 1 | 55 | 7 |
| Tefi nostat | 24 | 1 | 24 | 23 |
| Splitomicin | 5 | 0 | 5 | 1 |
| ACY-738 | 5 | 1 | 31 | 1 |
| Ricolinostat | 4 | 1 | 35 | 1 |
| J22352 | 5 | 0 | 3 | 2 |
| HPOB | 6 | 1 | 13 | 1 |
| MPT0G211 | 6 | 1 | 19 | 2 |
| NKL 22 | 7 | 0 | 13 | 1 |
| Tubastatin A | 5 | 1 | 6 | 1 |
| SR-4370 | 5 | 0 | 7 | 1 |
| Bufexamac | 4 | 1 | 50 | 0 |
| CG347B | 4 | 1 | 5 | 0 |
| Pyroxamide | 5 | 1 | 3 | 1 |
| MC1568 | 5 | 0 | 6 | 91 |
| Santacruzamate A | 3 | 2 | 3 | 1 |
| BML-210 | 7 | 1 | 19 | 1 |
| Tasquinimod | 7 | 0 | 4 | 0 |
| Pimelic Diphenylamide 106 | 4 | 1 | 17 | 2 |
| AES-350 | 5 | 1 | 10 | 1 |
| BRD 4354 | 4 | 0 | 10 | 3 |
| ACY-775 | 4 | 0 | 9 | 1 |
| Domatinostat | 4 | 5 | 7 | 8 |
| SW-100 | 4 | 0 | 0 | 2 |
| AES-135 | 4 | 1 | 1 | 6 |
| Sinapinic acid | 3 | 1 | 3 | 1 |
| Marein | 5 | 0 | 4 | 74 |
| Crotonoside | 3 | 0 | 3 | 1 |
| WT-161 | 1 | ND | 2 | ND |
| BRD73954 | 3 | 0 | 4 | 1 |
| Ac-Lys-AMC | 3 | 0 | 1 | 0 |
| KA2507 | 2 | 1 | 18 | 1 |
| RG2833 | 5 | 1 | 9 | 1 |
| ACY-1083 | 5 | 1 | 4 | 1 |
| PCI-34051 | 2 | 1 | 1 | 1 |
| BG45 | 4 | 1 | 5 | 2 |
| Tacedinaline | 2 | 1 | 4 | 1 |
| FT895 | 1 | 0 | 0 | 1 |
| Suberoyl bis-hydroxamic acid | 4 | 1 | 10 | 1 |
| TMP195 | 2 | 1 | 1 | 1 |
| Remetinostat | 3 | 0 | 2 | 1 |
| Tubastatin A (Hydrochloride) | 3 | 1 | 6 | 1 |
| Droxinostat | 3 | 0 | 4 | 2 |
| Gnetol | 2 | 1 | 3 | 1 |
| 1-Naphthohydroxamic acid | 3 | 0 | 2 | 0 |
| BRD3308 | 4 | 1 | 5 | 1 |
| UF010 | 3 | 0 | 2 | 1 |
| TMP269 | 3 | 0 | 3 | 1 |
| TH34 | 2 | 1 | 1 | 1 |
| RGFP966 | 1 | 1 | 2 | 1 |
| HDAC8-IN-1 | 2 | 0 | 0 | 2 |
| CHDI-390576 | 2 | 0 | 0 | 1 |
| ITSA-1 | 3 | 0 | 1 | 0 |
| Tubacin | ND | ND | 5 | 4 |

Accordingly, in some embodiments, the HDAC inhibitor is selected from the group consisting of: Oxamflatin, Quisinostat, Belinostat, Pracinostat, Trichostatin A, Dacinostat, Nanatinostat, LMK-235, Panobinostat, FNDR-20123, QTX125 (TFA), Givinostat (hydrochloride monohydrate), Fimepinostat, M344, Nampt-IN-3, HDACs/mTOR Inhibitor 1, ACY-957, CAY10603, BRD-6929, Mocetinostat, CUDC-101, Resminostat (hydrochloride), Corin, Scriptaid, CXD101, HDAC-IN-3, JAK/HDAC-IN-1, Citarinostat, Entinostat, Tucidinostat, Vorinostat, Nexturastat A, EDO-S101, Tefinostat, Splitomicin, ACY-738, Ricolinostat, J22352, HPOB, MPT0G211, NKL 22, Tubastatin A, SR-4370, Bufexamac, CG347B, Pyroxamide, MC1568, Santacruzamate A, BML-210, Tasquinimod, Pimelic Diphenylamide 106, AES-350, BRD 4354, ACY-775, Domatinostat, SW-100, AES-135, Sinapinic acid, Marein, Crotonoside, WT-161, BRD73954, Ac-Lys-AMC, KA2507, RG2833, ACY-1083, PCI-34051, BG45, Tacedinaline, FT895, Suberoyl bis-hydroxamic acid, TMP195, Remetinostat, Tubastatin A (Hydrochloride), Droxinostat, Gnetol, 1-Naphthohydroxamic acid, BRD3308, UF010, TMP269, TH34, RGFP966, HDAC8-IN-1, CHDI-390576 and ITSA-1. In some embodiments, the HDAC inhibitor is selected from the group consisting of: Quisinostat, Belinostat, Pracinostat, Nanatinostat, Panobinostat, Givinostat (hydrochloride monohydrate), Fimepinostat, Mocetinostat, CUDC-101, Resminostat (hydrochloride), CXD101, Citarinostat, Entinostat, Tucidinostat, Vorinostat, EDO-S101, Tefinostat, Ricolinostat, Domatinostat and KA2507.

In some embodiments, peroxisomal biogenesis disorder results from a mutation in a PEX gene. In some embodiments, the mutation is a missense mutation.

In some embodiments, the PEX gene is PEX1, PEX6, PEX12 or PEX26.

In some embodiments, the PEX gene is PEX1 and the mutation is p.Gly843Asp, p.Arg795* or a splice cite in the intron between exon 11 and 12.

In some embodiments, the PEX gene is PEX6 and the mutation is p.Gln828Ter, p.Ser310Leu, p.Arg601Gln or p.Arg812Gln.

In some embodiments, the PEX gene is PEX26 and the mutation is p.Arg98Trp, c.G255insT or Thr77fs139*.

HDAC inhibitors can be incorporated into pharmaceutical compositions. Pharmaceutical compositions can be prepared according to conventional methods. By way of example, pharmaceutical compositions containing a HDAC inhibitor can be prepared by mixing said inhibitor with one or several pharmaceutically acceptable excipients which are chosen depending on the form of preparation desired for administration. Suitable excipients include e.g. fillers, binders, disintegrating agents and lubricants. Suitable routes of administration include oral and parenteral (e.g. subcutaneous, intramuscular, intravenous) routes.

The present disclosure thus also relates to a method of preventing or treating a peroxisomal biogenesis disorder which comprises administering a histone deacetylase inhibitor, or a pharmaceutical composition containing such an inhibitor, to a subject in need thereof.

In some embodiments, the HDAC inhibitor is as defined above.

In some embodiments, the peroxisomal biogenesis disorder results from a mutation, such as a missense mutation, in a PEX gene.

In some embodiments, the PEX gene is PEX1, PEX6, PEX12 or PEX26.

The present disclosure is illustrated by the following, non-limiting, examples.

### EXAMPLES

### MATERIALS AND METHODS

### Cell culture

Patient skin fibroblast cell lines were cultured at 37°C, 5% CO₂ in a 50:50 High Glucose Dulbecco's Modified Eagle Medium : Ham's F10, supplemented with 10% Foetal bovine serum , 1% Penicillin-Streptomycin.

Primary PEX304 cells (PEX1 deficient) were expanded from skin biopsy in our laboratory (OPERANDO *NCT* 03763864).

PEX405 cells (PEX1 deficient) were obtained from CHRU Strasbourg-France.

PEX506 and PEX607 cells (PEX6 deficient) were obtained from Institut de Pathologie et de Génétique - Gosselies-Belgium.

Fibroblasts GM08398 (Healthy), GM16513 (PEX1 deficient), GM11335, GM16865 and GM17398 (PEX26 deficient) were obtained from Coriell Cell Repositories.

The mutations of cells and the corresponding affected gene are listed in Table 3.

**Table 3**

| **Cells** | **Gene affected** | **Mutations** |
|---|---|---|
| GM16513 | PEX1 | c.2383C>T(p.Arg795*) |
| | | c. 2528G >A(p.G ly843Asp) |
| PEX405 | PEX1 | c. 2528 G>A (p. Gly843Asp) |
| | | c. 2071+1 G>T (splice cite in Intron between exon 11 and 12) |
| PEX304 | PEX1 | c. 2528 G>A (p. Gly843Asp) |
| | | c. 3438+1 G>T (splice cite in Intron between exon 21 and 22) |
| PEX506 | PEX6 | c.2482C>T(p.Gln828Ter) |
| | | c.929C>T (p.Ser310Leu) |
| PEX607 | PEX6 | c.1802G>A (p.Arg601Gln) |
| | | c.2435G>A (p.Arg812Gln) |
| GM11335 | PEX26 | Homozygous for c.292C>T (p.Arg98Trp=R98W) |
| GM16865 | PEX26 | c.292C>T (p.Arg98Trp=R98W) |
| | | c.G255insT (c.Gly255insThr) resulting in a frameshift inducing a 28 amino acid sequence distinct from normal PEX26p |
| GM 17398 | PEX26 | Homozygous for c.230 +1,G>T (IVS2+1,G>T)] a mutation in the splice-donor site of intron 2 in the PEX26 gene [c.230 +1,G>T (IVS2+1,G>T)], which leads to a frameshift at codon 77 and premature termination (T77fs139X) |

### HDAC inhibitors

A library of 87 HDAC inhibitors was purchased from MedchemExpress as 10mM stock solutions in DMSO.

### Toxicity assay

The cytotoxicity of compounds was assed using a double nucleus staining with Hoechst 33342 at 2 µg/mL (all cells) and Propidium Iodide at 1µg/mL (necrotic cells) for 30 min at 37°C. Images were acquired using the EnSight multimode plate reader (PerkinElmer) and analysis was done using Columbus analysis software (PerkinElmer). DAPI channel was used to find nuclei (Hoechst 33342) and the % of nuclei stained with Propidium Iodide was calculated and defined as dead cells. Then the total number of cells and the % of dead cells was calculated.

### Immunofluorescence

Primary fibroblasts were seeded at 500-700 cells per well in 384-well plates and grown at 37°C overnight. Cells were treated with test compounds or 0.1% DMSO for 3 days. After 3 days and a wash with PBS, cells were fixed with 4% paraformaldehyde (Electron Microscopy Sciences) in PBS for 20 minutes. After 3 washes with PBS, the cells were permeabilized and blocked with 0.3% Triton X-100, 1% BSA in PBS for 30 minutes at room temperature followed by 3 washes with 1% BSA in PBS. The cells were incubated with a rabbit Catalase polyclonal antibody (#12980 Cell signaling) overnight at 4°C. Following 3 washes with PBS containing 0.1% Tween-20, the cells were incubated for 1 h at room temperature with a conjugated goat anti-rabbit secondary antibody Alexa Fluor 647 (Ab150083-Abcam). The cells were then washed 3 times with PBS and Hoechst 33342 (H3570-ThermoFisher Scientific) + HCS Cellmask Blue (H32720 - ThermoFisher Scientific) staining was performed at 0.1 µg/ml and 0.4 µg/mL respectively for 30 minutes at room temperature. The cells were finally washed 3 times with PBS and fluorescent images were acquired using the INCell Analyzer 6000 imaging platform (GE Healthcare Life Sciences).

### High-Content Screening and Data Analysis

Images analysis was done using Columbus analysis software (PerkinElmer). DAPI channel was used to find nuclei (Hoechst 33342) and cytoplasm (HCS CellMask Blue) and border objects were removed. Cy5 channel (AlexaFluor 647) was used to find spots corresponding to peroxisomes which correctly import catalase. Cells were classified as importing or non-importing cells using a linear classifier method after training with images of wells containing Healthy cells and PEX1 deficient cells. The number of importing or non-importing cells was calculated. Finally, results were expressed as % of importing cells.

### Biochemical Analysis

### Beta-oxidation of d4-C24:0

Degradation (to d4-C16:0) and elongation (to d4-C26:0 and d4-C28:0) of d4-VLCFA (very long chain fatty acid) in the cells was measured using d4-C24:0 as substrate (D-6145-CDN isotopes). D4-C24:0 was solubilized in culture medium using α-cyclodetrin, with a molar ratio of d4-C24:0:α-cyclodextrin 1:50. Cyclodextrins, which are cyclic oligosaccharides, are commonly used to solubilize fatty acids by complexation, for supplementation of cell culture media, and serve as vehicle to deliver fatty acids to the cells. Cyclodextrins have a polar surface and a hydrophobic cylindrical cavity that can bind and solubilize a wide variety of hydrophobic molecules, such as fatty acids, while remaining soluble in water. The assay was performed in T75 culture flasks for 3 or 10 days. The medium was replaced by fresh medium every 2-3 days and supplemented with d4-C24:0 for the last 3 days. After 3 or 10 days, cells were harvested and lysated in water (100-200 µL) by freeze/thaw cycle and sonication for 5 min. Total proteins were quantified using BCA assay and 100 µg of total proteins were used for each sample.

### Sample preparation for LC-MS/MS analysis:

After cell lysis, the samples were dried using a rotating evaporator (Genevac EZ-2, SP Scientific), set at 40°C for 2h under vacuum. After evaporation, a hydrolysis mix consisting of 90% acetonitrile and 10% NaOH 1N in water was added to the samples. The samples were incubated for 1h at room temperature under stirring (300 rpm). After hydrolysis, the samples were evaporated using a Genevac device, under vacuum, at 40°C for 2h.

Derivation of free fatty acids was done using a 2.5 mg/mL Na₂CO₃ solution in DMF and a 2.5 mg/mL TMAE (trimethylammoniumethyl) reagent solution in DMF. The solutions were mixed in a 1:1 ratio, and 200 µL of the mix was added to dry samples to be derived. For the derivation, the samples were incubated for 3h at 50°C. DMF was then evaporated using a Genevac^{™} device, under vacuum, at 60°C for 4h.

Before injection, the samples were solubilized in a 90/10 acetonitrile/isopropanol mixture with 1% HCl. After solubilization, the samples were stirred and centrifuged at 4000 rpm for 20 min at 10°C.

For the separation of the derived fatty acids by liquid chromatography, a Waters Acquity ultra-performance liquid chromatography (UPLC) system (Acquity I-Class, Waters) was used. A Waters Acquity UPLC BEH (Ethylene Bridged Hybrid) C8 column (2.1 × 50mm, particle size 1.7 µm, pore size 130 A, 2.1mm inner diameter) preceded by a metal pre-filter (KrudKatcher Ultra HPLC In-Line Filter, AF0-8497, Phenomenex) was used for separation. The column temperature was maintained at 40 °C. The injection volume was 3µL for each analysis. The mobile phase A was composed of acetonitrile:water 90:10 v/v containing 10mM ammonium formate pH 9.2 and the mobile phase B was 100% isopropanol. The separation was done using a gradient method, with a flow rate set at 0.7 mL/min throughout the separation:

| **Run time (min)** | **% B** |
|---|---|
| 0.00 | 5 |
| 0.40 | 5 |
| 0.50 | 30 |
| 0.90 | 30 |
| 1.00 | 5 |
| 1.60 | 5 |

After separation by liquid chromatography, the analytes were subjected to MS analysis using a Waters Xevo TQSmicro system (Waters). The analytes were ionized using positive electrospray ionization (positive ESI). The molecules were analyzed using MRM (multiple reaction monitoring) mode, with the main daughter ion for each derived fatty acid corresponding to the loss of a trimethylammonium moiety (m/z 59). Derived FAs with a chain length from 16 to 28, with no or one unsaturation, were analyzed. MS settings were as follows: capillary voltage, 2.5 kV; cone, 33 V; source temperature, 150°C; desolvation temperature, 600°C; cone gas flow, 100 L/h; desolvation gas flow, 1200 L/h. The acquired data were analyzed by the Waters MassLynx software.

### Plasmalogens synthesis

The assay was performed in T75 culture flasks for 3 or 10 days. The medium was replaced by fresh medium every 2-3 days. After 3 or 10 days, the cells were harvested and lysated in water (100-200 µL) by freeze/thaw cycle and sonication for 5 min. Total proteins were quantified using BCA assay and 100 µg of total proteins were used for each sample.

### Sample preparation for LC-MS/MS analysis

After cell lysis, the samples were dried using a rotating evaporator (Genevac EZ-2, SP Scientific), set at 40°C for 2h under vacuum. Then a solution containing water/acetonitrile 20/80 v/v was added to the wells. Before analysis, the samples were centrifuged at 4000 rpm for 20 min at room temperature. For the separation of plasmalogens by liquid chromatography, a Waters Acquity ultra-performance liquid chromatography (UPLC) system (Acquity I-Class, Waters) was used. A Waters Acquity UPLC BEH (Ethylene Bridged Hybrid) C8 column (2.1 × 50mm, particle size 1.7 µm, pore size 130 A, 2.1mm inner diameter) preceded by a metal pre-filter (KrudKatcher Ultra HPLC In-Line Filter, AF0-8497, Phenomenex) was used for separation. The column temperature was maintained at 50°C. The injection volume was 5µL for each analysis. The mobile phase A was composed of water + 0.1% formic acid and the mobile phase B was methanol + 0.1% formic acid. The separation was done using a gradient, with a flow rate set at 0.7 mL/min throughout the separation:

| **Run time (min)** | **% B** |
|---|---|
| 0.00 | 77 |
| 0.20 | 77 |
| 0.50 | 90 |
| 1.10 | 90 |
| 1.50 | 99 |
| 2.00 | 77 |

During analysis, the samples were kept at 10°C, and 5 µL of each sample were injected. After separation by liquid chromatography, the analytes were subjected to MS/MS analysis using a Waters Xevo TQSmicro system (Waters). The analytes were ionized using positive electrospray ionization (positive ESI). The molecules were analyzed using MRM mode. The analyzed plasmalogens contained moieties with C16:0, C18:0, C18:1 at the sn-1 position and fatty acid of chain-length 18 to 24 with 1 to 6 unsaturations at the sn-2 position. After LC-MS/MS analysis, the area under the peak of each cellular metabolite of interest was integrated. The cellular levels of plasmalogens were calculated using the area of each plasmalogen divided by protein quantity. For MS analysis, MS settings were as follows: capillary voltage, 2.5 kV; cone, 33 V; source temperature, 150°C; desolvation temperature, 600°C; cone gas flow, 100 L/h; desolvation gas flow, 1200 L/h. The acquired data were analyzed by the Waters MassLynx software.

### EXAMPLE 1: Screening of HDAC inhibitors library on GM16513 and PEX405 cells

All HDAC inhibitors were tested at 1.3 and 10 µM for 3 days on GM16513 and PEX405 cells. The cytotoxicity was assessed in parallel using a double nucleus staining with Hoechst 33342 and Propidium Iodide (PI). The % of importing cells and the % of PI positive cells (dead cells), determined following the procedure described in "Materials and methods", are shown in Tables 1 and 2 above.

### EXAMPLE 2: Drug response of HDAC inhibitors on GM16513 cells

Representative HDAC inhibitors were tested in a dose response manner with 6 concentrations (see figures 1A-1G). The EC50 and maximum efficacy of these inhibitors were determined. The results are shown in Table 4.

**Table 4**

| **Molecule name** | **EC50 (µM)** | **Max Efficacy (% Importing cells)** |
|---|---|---|
| Panobinostat | 0.003135 | 93 |
| Quisinostat | 0.03 | 95 |
| Nanatinostat | 0.05 | 93 |
| Pracinostat | 0.17 | 93 |
| Givinostat | 0.17 | 80 |
| Belinostat | 0.2 | 98 |
| Mocetinostat | 0.25 | 50 |
| CXD101 | 0.28 | 48 |
| Entinostat | 0.47 | 54 |
| Domatinostat | 0.6 | 33 |
| CUDC-101 | 0.7 | 100 |
| Vorinostat | 1.09 | 90 |
| Resminostat | 1.4 | 86 |
| Tucidinostat | 1.5 | 70 |
| Ricolinostat | 2.76 | 96 |
| EDO-S101 | 3 | 90 |
| Citarinostat | 3.2 | 93 |
| Tefinostat | 4 | 97 |
| KA2507 | 14.3 | 100 |
| Fimepinostat | 0.01 | 92.5 |

### EXAMPLE 3: Effect of Belinostat on the peroxisomal function

The effects of Belinostat (one of the most effective and potent HDAC inhibitor already approved for a human use) on peroxisomal biochemical function were investigated. Peroxisomal enzymatic activities are essential for the catabolism of very long chain fatty acids (VLCFA) and synthesis of ether glycerophospholipids plasmalogens. The relative levels of VLCFA and plasmalogens were first evaluated in GM16513 (PEX1 deficient) and GM08398 (Healthy control) cells in the absence of any treatment. Consistent with prior reports PEX1 deficient cells showed elevated VLCFA levels and a lower plasmalogens levels ^{2,3,22,23}. Belinostat treatment at 0.5 µM for 3 or 10 days resulted in a decrease in relative d4-VLCFA levels (C26, C28) and an increase in shortest d4-FA (C16, C18) levels in GM16513 cells represented with the fold change compared to non-treated cells in figure 2.

In addition to this effect on β-oxidation, Belinostat at 0.5 µM over 3 or 10 days increased plasmalogen levels in GM16513 cells as shown in figure 3.

Taken together these results show that Belinostat is capable to restore the peroxisomal function.

### EXAMPLE 4: Effect of a 3-day treatment with Givinostat on peroxisomal function

The effects of Givinostat, another HDAC inhibitor, on peroxisomal function were also studied after 3 days of treatment. Givinostat is currently in development for the treatment of Duchenne and Becker Muscular Dystrophy and has shown a good safety profile in clinical trials in children without important side effects (see e.g. study NCT01761292). In the above-mentioned clinical trials, Givinostat was administered orally. Givinostat could therefore represent an alternative to Belinostat especially in the paediatric population (since the route of administration of Belinostat - i.e., intravenous - is not always desired or suitable in children). As previously described for Belinostat in GM16513 cells, Givinostat treatment also resulted in a decrease in relative d4-C28, C26 FA levels and an increase in d4-C16,C18,C20 and C22 levels in GM16513 and PEX405 cells in a dose response manner as shown in figure 4. It can be seen that Givinostat has an effect on β-oxidation which is comparable to that of Belinostat. In addition Givinostat treatment over 3 days increased plasmalogen levels in GM16513 and PEX405 cells as shown in figure 5. Taken together these results show that Givinostat is capable to restore the peroxisomal function.

### EXAMPLE 5: Effect of HDAC inhibitors on PEX6 and PEX26 deficient cells

Among the HDAC inhibitors of example 2, 4 were tested on other cells which are PEX1, PEX6 or PEX26 deficient. Gene affected and mutations of all cells are listed in Table 3 above. Data (lower dose leading to the maximum effect) are presented in Table 5.

**Table 5**

| **Gene deficiency** | **Cells** | **Molecule Name** | **Concentration (µM)** | **Importing cells (%)** |
|---|---|---|---|---|
| PEX1 | PEX405 | DMSO 0.3% | / | 2 |
| | | Belinostat | 2.5 | 41 |
| | | Givinostat | 0.25 | 13 |
| | | Vorinostat | 10 | 16 |
| | | Ricolinostat | 10 | 27 |
| | PEX304 | DMSO 0.3% | / | 1 |
| | | Belinostat | 2.5 | 11 |
| | | Givi nostat | 0.85 | 5 |
| | | Vorinostat | 3 | 8 |
| | | Ricolinostat | 10 | 5 |
| PEX6 | PEX506 | DMSO 0.3% | / | 42 |
| | | Belinostat | 0.25 | 64 |
| | | Givinostat | 0.087 | 69 |
| | | Vorinostat | 0.33 | 57 |
| | | Ricolinostat | 0.33 | 55 |
| | PEX607 | DMSO 0.3% | / | 19 |
| | | Belinostat | 0.85 | 43 |
| | | Givinostat | 0.25 | 42 |
| | | Vorinostat | 3 | 48 |
| | | Ricolinostat | 3 | 40 |
| PEX26 | GM11335 | DMSO 0.3% | / | 6 |
| | | Belinostat | 0.85 | 30 |
| | | Givinostat | 0.087 | 20 |
| | | Vorinostat | 3 | 25 |
| | | Ricolinostat | 10 | 18 |
| | GM 16865 | DMSO 0.3% | / | 8 |
| | | Belinostat | 0.85 | 35 |
| | | Givinostat | 0.25 | 20 |
| | | Vorinostat | 3 | 27 |
| | | Ricolinostat | 10 | 20 |
| | GM 17398 | DMSO 0.3% | / | 0 |
| | | Belinostat | 2.5 | 0 |
| | | Givinostat | 2.5 | 0 |
| | | Vorinostat | 30 | 2 |
| | | Ricolinostat | 30 | 0 |

It can be noted that the percentage of cells which import at least a part of catalase in peroxisomes is different depending on gene deficiency and mutations. Interestingly all PEX1 deficient cells that were used shared the common mutation p.G843D in one allele. The second mutation present in GM16513 cells is a nonsense mutation whereas PEX304 cells and PEX405 cells respectively carry a mutation in a splice cite in intron between exon 21 and exon 22 or between exon 11 and exon 12. This identity of the second mutation seems to impact the basal catalase import and the response to compounds.

Furthermore, an increase of catalase import was observed in cells with mutations in PEX6 and PEX26 deficient cells.

The absence of response of GM17398 cells can be explained by the presence of mutations which lead to a frameshift and a premature termination. Indeed, without wishing to be bound by theory, the suspected mechanism of action of HDAC inhibitors is the increase in stability of proteins by acting on HSP expression or by autophagy inhibition.

### REFERENCES

1. Zhang, R. et al. Recovery of PEX1-Gly843Asp peroxisome dysfunction by small molecule compounds. Proceedings of the National Academy of Sciences of the United States of America 107, 5569-5574 (2010).
2. MacLean, G. E. et al. Zellweger spectrum disorder patient-derived fibroblasts with the PEX1-Gly843Asp allele recover peroxisome functions in response to flavonoids. J Cell Biochem 120, 3243-3258 (2019).
3. Berendse, K. et al. Arginine improves peroxisome functioning in cells from patients with a mild peroxisome biogenesis disorder. Orphanet J Rare Dis 8, 138 (2013).
4. Ebberink, M. S. et al. Genetic classification and mutational spectrum of more than 600 patients with a Zellweger syndrome spectrum disorder. Hum. Mutat. 32, 59-69 (2011).
5. Crane, D. I., Maxwell, M. A. & Paton, B. C. PEX1 mutations in the Zellweger spectrum of the peroxisome biogenesis disorders. Hum. Mutat. 26, 167-175 (2005).
6. Argyriou, C., D'Agostino, M. D. & Braverman, N. Peroxisome biogenesis disorders. Transl Sci Rare Dis 1, 111-144 (2016).
7. Fiedorczuk, K. & Chen, J. Mechanism of CFTR correction by type I folding correctors. Cell 185, 158-168.e11 (2022).
8. Alfonso, P. et al. Miglustat (NB-DNJ) works as a chaperone for mutated acid beta-glucosidase in cells transfected with several Gaucher disease mutations. Blood Cells Mol. Dis. 35, 268-276 (2005).
9. Porto, C. et al. The Pharmacological Chaperone N-butyldeoxynojirimycin Enhances Enzyme Replacement Therapy in Pompe Disease Fibroblasts. Molecular Therapy 17, 964-971 (2009).
10. Kirkegaard, T. et al. Heat shock protein-based therapy as a potential candidate for treating the sphingolipidoses. Sci. Transl. Med. 8, (2016).
11. Fog, C. K. et al. The heat shock protein amplifier arimoclomol improves refolding, maturation and lysosomal activity of glucocerebrosidase. EBioMedicine 38, 142-153 (2018).
12. Maegawa, G. H. B. et al. Identification and Characterization of Ambroxol as an Enzyme Enhancement Agent for Gaucher Disease. Journal of Biological Chemistry 284, 23502-23516 (2009).
13. Hutt, D. M. et al. Reduced histone deacetylase 7 activity restores function to misfolded CFTR in cystic fibrosis. Nat Chem Biol 6, 25-33 (2010).
14. Anglès, F., Hutt, D. M. & Balch, W. E. HDAC inhibitors rescue multiple disease-causing CFTR variants. Human Molecular Genetics 28, 1982-2000 (2019).
15. Lu, J. et al. Histone deacetylase inhibitors prevent the degradation and restore the activity of glucocerebrosidase in Gaucher disease. Proc. Natl. Acad. Sci. U.S.A. 108, 21200-21205 (2011).
16. Pipalia, N. H. et al. Histone deacetylase inhibitors correct the cholesterol storage defect in most Niemann-Pick C1 mutant cells. Journal of Lipid Research 58, 695-708 (2017).
17. Angelini, C. I., Piluso, G. & Nissan, X. Dual Blockade of Misfolded Alpha-Sarcoglycan Degradation by Bortezomib and Givinostat Combination. Frontiers in Pharmacology 13, 18 (2022).
18. Bartoli, M. et al. Mannosidase I inhibition rescues the human α-sarcoglycan R77C recurrent mutation. Human Molecular Genetics 17, 1214-1221 (2008).
19. Dranchak, P. K. et al. Nonsense suppressor therapies rescue peroxisome lipid metabolism and assembly in cells from patients with specific PEX gene mutations. J. Cell. Biochem. 112, 1250-1258 (2011).
20. Wei, H., Kemp, S., McGuinness, M. C., Moser, A. B. & Smith, K. D. Pharmacological induction of peroxisomes in peroxisome biogenesis disorders. Annals of Neurology 47, 286-296 (2000).
21. Ecker, J., Witt, O. & Milde, T. Targeting of histone deacetylases in brain tumors. CNS Oncology 2, 359-376 (2013).
22. Imamura, A. et al. Temperature-Sensitive Mutation in PEX1 Moderates the Phenotypes of Peroxisome Deficiency Disorders. Hum Mol Genet 7, 2089-2094 (1998).
23. Waterham, H. R., Ferdinandusse, S. & Wanders, R. J. A. Human disorders of peroxisome metabolism and biogenesis. Biochimica et Biophysica Acta (BBA) - Molecular Cell Research 1863, 922-933 (2016).

## Claims

1. A histone deacetylase (HDAC) inhibitor, or a pharmaceutical composition containing such an inhibitor, for use in the prevention or treatment of a peroxisomal biogenesis disorder.

2. The HDAC inhibitor or pharmaceutical composition for the use of claim 1, wherein said inhibitor is selected from the group consisting of: Oxamflatin, Quisinostat, Belinostat, Pracinostat, Trichostatin A, Dacinostat, Nanatinostat, LMK-235, Panobinostat, FNDR-20123, QTX125 (TFA), Givinostat (hydrochloride monohydrate), Fimepinostat, M344, Nampt-IN-3, HDACs/mTOR Inhibitor 1, ACY-957, CAY10603, BRD-6929, Mocetinostat, CUDC-101, Resminostat (hydrochloride), Corin, Scriptaid, CXD101, HDAC-IN-3, JAK/HDAC-IN-1, Citarinostat, Entinostat, Tucidinostat, Vorinostat, Nexturastat A, EDO-S101, Tefinostat, Splitomicin, ACY-738, Ricolinostat, J22352, HPOB, MPT0G211, NKL 22, Tubastatin A, SR-4370, Bufexamac, CG347B, Pyroxamide, MC1568, Santacruzamate A, BML-210, Tasquinimod, Pimelic Diphenylamide 106, AES-350, BRD 4354, ACY-775, Domatinostat, SW-100, AES-135, Sinapinic acid, Marein, Crotonoside, WT-161, BRD73954, Ac-Lys-AMC, KA2507, RG2833, ACY-1083, PCI-34051, BG45, Tacedinaline, FT895, Suberoyl bis-hydroxamic acid, TMP195, Remetinostat, Tubastatin A (Hydrochloride), Droxinostat, Gnetol, 1-Naphthohydroxamic acid, BRD3308, UF010, TMP269, TH34, RGFP966, HDAC8-IN-1, CHDI-390576 and ITSA-1.

3. The HDAC inhibitor or pharmaceutical composition for the use of claim 1, wherein said inhibitor is selected from the group consisting of: Quisinostat, Belinostat, Pracinostat, Nanatinostat, Panobinostat, Givinostat (hydrochloride monohydrate), Fimepinostat, Mocetinostat, CUDC-101, Resminostat (hydrochloride), CXD101, Citarinostat, Entinostat, Tucidinostat, Vorinostat, EDO-S101, Tefinostat, Ricolinostat, Domatinostat and KA2507.

4. The HDAC inhibitor or pharmaceutical composition for the use of any preceding claim, wherein the peroxisomal biogenesis disorder results from a mutation in a PEX gene.

5. The HDAC inhibitor or pharmaceutical composition for the use of claim 4, wherein the PEX gene is PEX1, PEX6, PEX12 or PEX26.

6. The HDAC inhibitor or pharmaceutical composition for the use of claim 4 or claim 5, wherein the mutation is a missense mutation.

7. The HDAC inhibitor or pharmaceutical composition for the use of claim 4 or claim 5, wherein the PEX gene is PEX1 and the mutation is p.Gly843Asp, p.Arg795* or a splice cite in the intron between exon 11 and 12.

8. The HDAC inhibitor or pharmaceutical composition for the use of claim 4 or claim 5, wherein the PEX gene is PEX6 and the mutation is p.Gln828Ter, p.Ser310Leu, p.Arg601Gln or p.Arg812Gln.

9. The HDAC inhibitor or pharmaceutical composition for the use of claim 4 or claim 5, wherein the PEX gene is PEX26 and the mutation is p.Arg98Trp, c.G255insT or Thr77fs139*.

10. A method of preventing or treating peroxisomal biogenesis disorder which comprises administering a histone deacetylase inhibitor, or a pharmaceutical composition containing such an inhibitor, to a subject in need thereof.

11. The method of claim 10, wherein said inhibitor is selected from the group consisting of: Oxamflatin, Quisinostat, Belinostat, Pracinostat, Trichostatin A, Dacinostat, Nanatinostat, LMK-235, Panobinostat, FNDR-20123, QTX125 (TFA), Givinostat (hydrochloride monohydrate), Fimepinostat, M344, Nampt-IN-3, HDACs/mTOR Inhibitor 1, ACY-957, CAY10603, BRD-6929, Mocetinostat, CUDC-101, Resminostat (hydrochloride), Corin, Scriptaid, CXD101, HDAC-IN-3, JAK/HDAC-IN-1, Citarinostat, Entinostat, Tucidinostat, Vorinostat, Nexturastat A, EDO-S101, Tefinostat, Splitomicin, ACY-738, Ricolinostat, J22352, HPOB, MPT0G211, NKL 22, Tubastatin A, SR-4370, Bufexamac, CG347B, Pyroxamide, MC1568, Santacruzamate A, BML-210, Tasquinimod, Pimelic Diphenylamide 106, AES-350, BRD 4354, ACY-775, Domatinostat, SW-100, AES-135, Sinapinic acid, Marein, Crotonoside, WT-161, BRD73954, Ac-Lys-AMC, KA2507, RG2833, ACY-1083, PCI-34051, BG45, Tacedinaline, FT895, Suberoyl bis-hydroxamic acid, TMP195, Remetinostat, Tubastatin A (Hydrochloride), Droxinostat, Gnetol, 1-Naphthohydroxamic acid, BRD3308, UF010, TMP269, TH34, RGFP966, HDAC8-IN-1, CHDI-390576 and ITSA-1.

12. The method of claim 10, wherein said inhibitor is selected from the group consisting of: Quisinostat, Belinostat, Pracinostat, Nanatinostat, Panobinostat, Givinostat (hydrochloride monohydrate), Fimepinostat, Mocetinostat, CUDC-101, Resminostat (hydrochloride), CXD101, Citarinostat, Entinostat, Tucidinostat, Vorinostat, EDO-S101, Tefinostat, Ricolinostat, Domatinostat and KA2507.

13. The method of any one of claims 10 to 12, wherein the peroxisomal biogenesis disorder results from a mutation, such as a missense mutation, in a PEX gene.

14. The method of claim 13, wherein the PEX gene is PEX1, PEX6, PEX12 or PEX26.
